# EUROPEAN PATENT APPLICATION

(11) **EP 4 008 390 A1**
(43) Date of publication of application: **08.06.2022**
(21) Application number: 20849519.2
(22) Date of filing: 17.01.2020
(51) Int. Cl.: A61M 25/10

(54) **DRUGCOATED BALLOON CONTROLLABLE IN DRUG METABOLISM AND PREPARATION METHOD THEREFOR**

(30) Priority: 02.08.2019 CN 201910711649
(71) Applicant: Shanghai Heartcare Medical Technology Corporation Limited, Shanghai 201201 (CN)
(72) Inventor: WANG, Guohui, Shanghai 201201 (CN); ZHANG, Chenzhao, Shanghai 201201 (CN); WANG, Junyi, Shanghai 201201 (CN)
(74) Representative: Mewburn Ellis LLP
(86) International application number: PCT/CN2020/072696
(87) International publication number: WO 2021/022799

(57) **Abstract**

The present invention provides a preparation method for a drug-coated balloon controllable in drug metabolism. A drug receptor protein inhibitor is mixed with drugs, water and ethanol to obtain a medicinal liquid, the medicinal liquid is sprayed onto the surface of a balloon back and forth by means of an ultrasonic spraying device, and drying is performed so as to prepare the drug-coated balloon. The drug receptor protein inhibitor can also be replaced with other agents such as a drug metabolism isoenzyme inhibitor, a drug metabolism isoenzyme inducer, or a mixture of the drug receptor protein inhibitor and the drug metabolism isoenzyme inhibitor for preparing the medicinal liquid, and a drug metabolic cycle of the drug-coated balloon can be further adjusted and controlled by adjusting the ratio of the addition quantity of the drug receptor protein inhibitor or the other agents to the drugs. The drug-coated balloon prepared by the method has the functions that effective controllable drug metabolism can be implemented without destroying the structure of an intima, the drug metabolism can be implemented to achieve the effect of drug treatment at an early stage of using the drug-coated balloon, and the effect period of the drugs can be prolonged by adjusting the ratio.

## Description

### Field of the Invention

The present invention relates to the technical field of drug-coated balloons, in particular to a drug-coated balloon controllable in drug metabolism and preparation method therefor.

### Description of the Prior art

The drug-coated balloon is a catheter-based drug delivery device. This concept was proposed by Harvey Wolinsky in 1991 to prevent vascular restenosis after percutaneous transluminal angioplasty. Its mechanism of action is to inhibit intimal hyperplasia by carrying drugs. When the drugs coated on the surface of the balloon reaches the lesion, the balloon expands the lesion by pressurizing the balloon, and at the same time, the coated drugs are continuously released here, so that the vascular wall can fully absorb the drugs and inhibit the occurrence of restenosis. The drug-coated balloon method does not require radiotherapy, polymer or other sustained release technologies, and can deliver drugs to all areas within the reach of the balloon.

Drug-coated balloon is a novel therapeutic technology which uses balloon for drug release and is developed on the basis of interventional techniques such as percutaneous transluminal angioplasty or balloon angioplasty. The therapeutic technology is to coat anti-proliferative drugs, such as paclitaxel, on the surface of a balloon, and when the balloon reaches the lesion of the vascular wall and is stretched, expanded, and contacted with the intima of the vascular wall, the drugs can be quickly released and transferred into the local vascular wall by tearing the intima of the vascular wall and pressurizing the balloon. The drugs play a role in local anti-vascular intimal hyperplasia, thereby preventing vascular restenosis after vascular intervention. There are three ways for drugs to enter the vascular tissue: 1. the balloon inflates and the drugs are released from the coating surface and quickly dissolved and enter the intima cells under the action of a cosolvent; 2. the balloon inflates and tears a part of the intima, and the drugs fall off the surface coating of the balloon and enter the torn gap, thereby achieving a long-term and slow release of drug;3. the drugs contact with the vascular wall tissue, contact with the lipophilic site of the cell, and adhere to the cell wall so that it is difficult for the blood to wash away the drugs, thereby achieving a long-term release of drug.

Chinese invention patent CN 201310594716 discloses a method for preparing a drug-coated balloon. The patented method for slowing down drug metabolism requires the balloon to be inflated to a larger diameter to the extent of tearing the intima. However, this method itself is a kind of damage to the blood vessels and in the later healing process, the site of lesion often stimulates cell growth which forms a thicker intima. Chinese invention patent CN 201220483575 discloses an arsenic trioxide-coated balloon controllable in release of drugs. A sustained-release layer of arsenic trioxide is coated on the surface of the balloon by taking advantage of the rapid water solubility of arsenic trioxide. In the sustained-release layer, uniform micro-cavities are formed to inhibit the metabolism of arsenic trioxide. In the balloon inflation process, the cavities are expanded into micropores by adjusting the size of the cavities, so that arsenic trioxide is rapidly metabolized at the vascular wall and uniformly enriched on the vascular wall surface to inhibit cell proliferation by using the rapid phagocytosis of cells, thereby achieving the effect of inhibiting restenosis. The patent uses the microporous structure in the sustained-release layer to reduce the contact area between the drugs and the intima to achieve the effect of sustained-release. This method adds a sustained-release layer. In the early stage, the sustained-release layer needs to be dissolved first. During this period, there is no drug release, that is, there is no drug effect in the early postoperative period, which would limit the therapeutic effect of the drug-coated balloon.

Therefore, those skilled in the art are committed to developing a drug-coated balloon controllable in drug metabolism and providing a simple and efficient preparation method for preparing the drug-coated balloon. The drug-coated balloon has the advantages that effective controllable drug metabolism can be implemented without destroying the structure of an intima and the drug can be released to achieve the effect at an early stage of using the drug-coated balloon. Meanwhile, the effect period of the drugs can be prolonged.

### Summary of the Invention

In view of the above-mentioned shortcomings of the prior art, the technical problem to be solved by the present invention is how to provide a drug-coated balloon controllable in drug metabolism and preparation method therefor. The prepared drug-coated balloon has the advantages that effective controllable drug metabolism can be implemented without destroying the structure of an intima and the drug can be released to achieve the effect of drug treatment at an early stage of using the drug-coated balloon and the effect period of the drugs can be increased.

In order to achieve the above objective, the present invention provides a method for preparing a drug-coated balloon controllable in drug metabolism, the method includes the following steps:
step 1: mixing a drug receptor protein inhibitor, a drug, water, and ethanol to prepare a medicinal liquid;
step 2: loading the medicinal liquid prepared in the step 1 into an ultrasonic spraying equipment, and setting flow parameters and a rotating speed of the balloon;
step 3: adjusting a distance between an ultrasonic nozzle of the ultrasonic spraying equipment and the balloon, controlling an ambient temperature at 18-28 °C, and wetting a surface of the balloon with ethanol before spraying;
step 4: turning on the ultrasonic spraying equipment and spraying back and forth along an axial direction of the balloon; and
step 5: drying the sprayed balloon to obtain the drug-coated balloon.

Further, the drug receptor protein inhibitor in the step 1 can be replaced by a drug metabolism isoenzyme inhibitor or a drug metabolism isoenzyme inducer.

Further, the drug receptor protein inhibitor in the step 1 can also be mixed for use with the drug metabolism isoenzyme inhibitor.

Further, the drug is rapamycin, and the drug receptor protein inhibitor is selected from one or more of inhibitors of immunoaffinity protein FKBP12: 3-pyridin-3-ylpropyl-(2S)-1-(3,3-dimethyl-2-oxo-pentanoyl)-pyrrolidine-2-carboxylat e, (3R)-4-(p-Toluenesulfonyl)-1,4-thiazane-3-carboxylicacid-L-leucine ethyl ester and (3R)-4-(p-Toluenesulfonyl)-1,4-thiazane-3-carboxylic acid-L-phenyla-lanine benzyl ester.

Further, the drug is rapamycin, and the drug metabolism isoenzyme inhibitor is selected from an inhibitor of CYP3A4 isoenzyme: one or more of posaconazole, erythromycin, telithromycin, HIV protease inhibitor, popravir, telaprevir, amiodarone, amprenavir, aprepitant, atonavir, cimetidine, ciprofloxacin, clarithromycin, diltiazem, doxycycline, enoxacin, fluconazole, fluvoxamine, imatinib, indinavir, itraconazole, ketoconazole, miconazole, nefazodone, ritonavir, saquinavir, telithromycin, verapamil, and voriconazole.

Further, the drug is rapamycin, and the drug metabolism isoenzyme inducer is selected from an inducer of CYP3A4 isoenzyme: one or more of aprepitant, barbiturates, bosentan, carbamazepine, efavirenz, felbamate, glucocorticoids, modafinil, nevirapine, oxcarbazepine, phenytoin, phenobarbital, primidone, etravirine, rifampicin, St. John's wort, pioglitazone and topiramate.

Further, a ratio of the drug receptor protein inhibitor to the drug is 0.25-4 : 1, and the drug metabolism slows down as a function of increase of an addition amount of the drug receptor protein inhibitor.

Further, a ratio of the drug metabolism isoenzyme inhibitor to the drug is 0.25-4 : 1, and the drug metabolism slows down as a function of increase of an addition amount of the drug metabolism isoenzyme inhibitor.

Further, a ratio of the drug metabolism isoenzyme inducer to the drug is 0.25-4 : 1, and the drug metabolism slows down as a function of increase of an addition amount of the drug metabolism isoenzyme inducer.

The present invention also provides a drug-coated balloon controllable in drug metabolism prepared according to the method for preparing a drug-coated balloon controllable in drug metabolism as described above. The drug-coated balloon can regulate a drug metabolic rate under a condition that a total drug load remains unchanged.

Compared with the prior art, the present invention has at least the following beneficial technical effects:
(1) the present invention realizes slow metabolism of drugs by adding a drug receptor protein inhibitor or a drug metabolism isoenzyme inhibitor, and does not need to inflate the balloon to a larger diameter to tear the intima to achieve slow drug metabolism, which reduces further damage to blood vessels and other tissues while ensuring the therapeutic effect by slowing down drug metabolism;
(2) the present invention can also achieve the purpose of accelerating drug metabolism by adding a drug metabolism isoenzyme inducer;
(3) the present invention can adjust the metabolic rate of drugs according to actual needs and increase the period of effect of the drugs by adjusting the amount of a drug receptor protein inhibitor or a drug metabolism isoenzyme inhibitor or inducer added under the condition that the total drug amount remains unchanged; and
(4) the drug-coated balloon controllable in drug metabolism provided by the present invention does not have a waiting period caused by the dissolution of the sustained-release layer. After the drug-coated balloon is introduced into body after surgery, the slow and controllable release of the drugs can be started, and the therapeutic effect of the drugs can be exerted.

In the following, the concept, specific structure and technical effects of the present invention will be further explained in conjunction with the accompanying drawings to fully understand the purpose, features and effects of the present invention.

### Brief Description of the Drawings

Figure 1 is a schematic diagram of spraying according to a preferred embodiment of the present invention;
Figure 2 is a flow chart of preparing a drug-coated balloon according to a preferred embodiment of the present invention;
Figure 3 is a schematic diagram of the active site of the drug receptor protein FBKP12 used in a preferred embodiment of the present invention;
Figure 4 is an inhibitor 3-pyridin-3-ylpropyl-(2S)-1-(3,3-dimethyl-2-oxo-pentanoyl)-pyrrolidine-2-carboxylat e of the drug receptor protein FBKP12 used in a preferred embodiment of the present invention;
Figure 5 is an inhibitor (3R)-4-(p-Toluenesulfonyl)-1,4-thiazane-3-carboxylicacid-L-leucine ethyl ester of the drug receptor protein FBKP12 used in a preferred embodiment of the present;
Figure 6 is an inhibitor (3R)-4-(p-Toluenesulfonyl)-1,4-thiazane-3-carboxylic acid-L-phenyla-lanine benzyl ester of the drug receptor protein FBKP12 used in a preferred embodiment of the present invention.

### Detailed Description of the Preferred Embodiments

Hereinafter, a number of preferred examples of the present invention will be introduced with reference to the drawings in the specification to make the technical content clearer and easier to understand. The present invention can be embodied by many different forms of examples, and the protection scope of the present invention is not limited to the examples mentioned in the text.

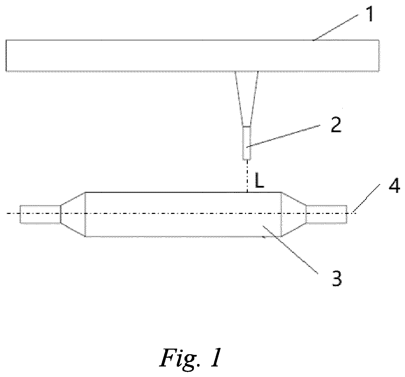

In the following examples, rapamycin is selected as the drug, and a schematic diagram of spraying the drug-coated balloon is shown in Figure 1. In Figure 1, the ultrasonic spraying equipment includes a nozzle rail 1 and an ultrasonic nozzle 2 arranged on the nozzle rail 1 and moving along the nozzle rail 1. The distance between the ultrasonic nozzle 2 and the balloon 3 is L, and the ultrasonic nozzle 2 moves back and forth along the direction of the axis 4 of the balloon 3 for spraying.

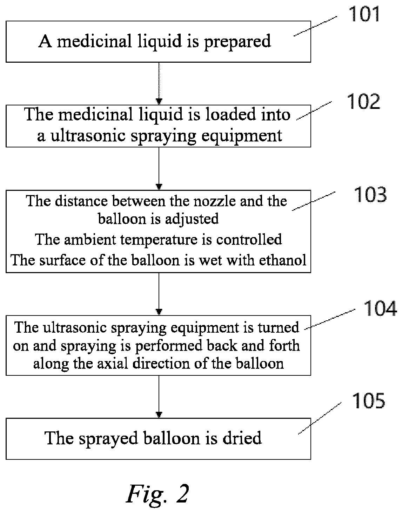

As shown in Figure 2, the method for preparing a drug-coated balloon is shown and includes:
step 1 101: formulating the medicinal liquid that needs to be sprayed on the balloon , which will be described in detail in the following content;
step 2 102: loading the medicinal liquid prepared in the step 1 into an ultrasonic spraying equipment, and setting flow parameters and a rotating speed of a balloon;
step 3 103: adjusting a distance L between the ultrasonic nozzle 2 of the ultrasonic spraying equipment and the balloon 3, controlling an ambient temperature at 18-28 °C, and wetting a surface of the balloon 3 with ethanol before spraying;
step 4 104: turning on the ultrasonic spraying equipment and spraying back and forth along the direction of the axis 4 of the balloon 3; and
step 5 105: drying the sprayed balloon 3 to obtain the drug-coated balloon.

### I. A drug receptor protein inhibitor was used to prepare the drug-coated balloon controllable in drug metabolism

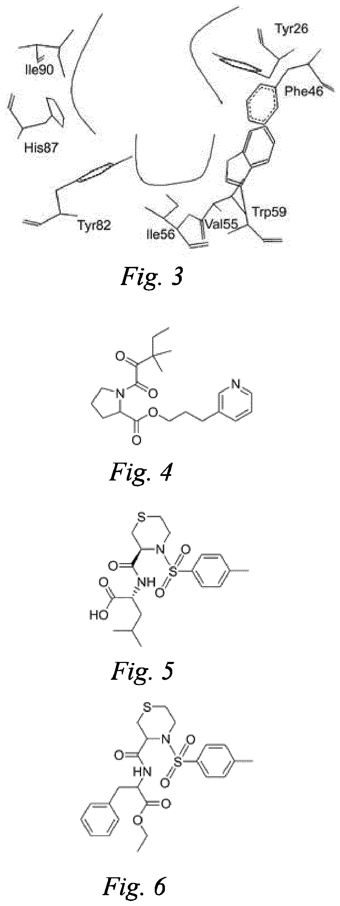

Rapamycin blocks signal transduction and blocks the progression of T lymphocytes and other cells from G1 to S phase through different cytokine receptors. Rapamycin can block the calcium-dependent and calcium-independent signal transduction pathways of T lymphocytes and B lymphocytes. Rapamycin can bind to the immunophilin FKBP12 to form a RAPA-FKBP12 complex, which cannot bind to calmodulin, and rapamycin does not inhibit the early activation of T cells or directly reduce the synthesis of cytokines. The FKBP12 protein mainly forms hydrogen bonds with the inhibitor through Ile56 and form a hydrophobic interaction zone through Tyr82, and Tyr26, Phe46, Val55, Tyr59, His87 and Ile90 (the active sites of the FKBP12 protein are shown in Figure 3). The binding of the inhibitor to FKBP12 can effectively slow down the binding rate of rapamycin to FKBP12 to extend the drug metabolism of the drug-coated balloon. Therefore, in the present invention, the purpose of extending the drug metabolism of the drug balloon is achieved by adding a inhibitor of rapamycin's receptor protein FKBP12, which binds to the FKBP12 in advance.

The main inhibitors of FKBP12 protein are 3-pyridin-3-ylpropyl-(2S)-1-(3,3-dimethyl-2-oxo-pentanoyl)-pyrrolidine-2-carboxylat e (abbreviated as drug a), (3R)-4-(p-Toluenesulfonyl)-1,4-thiazane-3-carboxylicacid-L-leucine ethyl ester (abbreviated as drug b), and (3R)-4-(p-Toluenesulfonyl)-1,4-thiazane-3-carboxylic acid-L-phenyla-lanine benzyl ester (abbreviated as drug c) (molecular structure diagrams are shown in Figures 4, 5, and 6 respectively). The ability to bind FKBP12 protein from strong to weak is drug c, drug b, and drug a. Therefore, in the following examples, the type and content of FKBP12 protein inhibitors added can be adjusted to regulate rapamycin metabolism.

### Example 1

The drug-coated balloon was prepared according to the following ratio and method:
1. FKBP12 inhibitor drug a (3%), rapamycin (1%), water (5-10%) and ethanol (82-93%) were mixed by weight ratio to obtain a medicinal liquid with rapamycin concentration at 52 mg/ml;
2. The medicinal liquid was loaded into an ultrasonic spraying equipment, and the flow rate was set to 0.01-0.1 ml/min;
3. The rotating speed of the balloon was set to 3 to 5.0 rev/s, and the surface of the balloon 3 was wet with ethanol before spraying;
4. The distance L between the ultrasonic nozzle 2 of the ultrasonic spraying equipment and the balloon 3 was adjusted to 10-30 mm, and the ambient temperature was controlled at 18-28 °C;
5. The ultrasonic spraying equipment was turned on, spraying was performed back and forth 10 times along the axial direction of the balloon 3, and the final drug content on the balloon 3 was controlled to 4.0 ug/mm²;
6. The sprayed balloon 3 was placed in an environment of 18-28 °C and dried for 120 minutes.

### Example 2

The drug-coated balloon was prepared according to the following ratio and method:
1. FKBP12 inhibitor drug c (4%), rapamycin (4%), water (5-10%) and ethanol (82-93%) were mixed by weight ratio to obtain a medicinal liquid with rapamycin concentration at 52 mg/ml;
2. The medicinal liquid was loaded into an ultrasonic spraying equipment, and the flow rate was set to 0.01-0.1 ml/min;
3. The rotating speed of the balloon was set to 3 to 5.0 rev/s, and the surface of the balloon 3 was wet with ethanol before spraying;
4. The distance L between the ultrasonic nozzle 2 of the ultrasonic spraying equipment and the balloon 3 was adjusted to 10-30 mm, and the ambient temperature was controlled at 18-28 °C;
5. The ultrasonic spraying equipment was turned on, spraying was performed back and forth 10 times along the axial direction of the balloon 3, and the final drug content on the balloon 3 was controlled to 4.0 ug/mm²;
6. The sprayed balloon 3 was placed in an environment of 18-28 °C and dried for 120 minutes.

### Example 3

The drug-coated balloon was prepared according to the following ratio and method:
1. FKBP12 inhibitor drug c (5%), rapamycin (1.25%), water (5-10%) and ethanol (82-93%) were mixed by weight ratio to obtain a medicinal liquid with rapamycin concentration at 52 mg/ml;
2. The medicinal liquid was loaded into an ultrasonic spraying equipment, and the flow rate was set to 0.01-0.1 ml/min;
3. The rotating speed of the balloon was set to 3 to 5.0 rev/s, and the surface of the balloon 3 was wet with ethanol before spraying;
4. The distance L between the ultrasonic nozzle 2 of the ultrasonic spraying equipment and the balloon 3 was adjusted to 10-30 mm, and the ambient temperature was controlled at 18-28 °C;
5. The ultrasonic spraying equipment was turned on, spraying was performed back and forth 10 times along the axial direction of the balloon 3, and the final drug content on the balloon 3 was controlled to 4.0 ug/mm²;
6. The sprayed balloon 3 was placed in an environment of 18-28 °C and dried for 120 minutes.

### II. A drug metabolism isoenzyme inhibitor or a drug metabolism isoenzyme inducer was used to prepare the drug-coated balloon controllable in drug metabolism

Rapamycin can be extensively metabolized by a CYP3A4 isoenzyme, so the absorption and the elimination of rapamycin after systemic absorption can be affected by a drug acting on this isoenzyme. An inhibitor of CYP3A4 can slow down the metabolism of rapamycin and increase the blood level of rapamycin; and an inducer of CYP3A4 can accelerate the metabolism of rapamycin and decrease the blood level.

Therefore, the present invention controls the metabolic rate of rapamycin by adding an inhibitor or an inducer of CYP3A4 isoenzyme, so as to achieve the purpose of controlling the drug metabolism of the drug-coated balloon.

Among them, the inhibitor of CYP3A4 can be selected from: one or more of posaconazole, erythromycin, telithromycin, HIV protease inhibitor, popravir, telaprevir, amiodarone, amprenavir, aprepitant, atonavir, cimetidine, ciprofloxacin, clarithromycin, diltiazem, doxycycline, enoxacin, erythrocin, fluconazole, fluvoxamine, imatinib, indinavir, itraconazole, ketoconazole, miconazole, nefazodone, ritonavir, saquinavir, telithromycin, verapamil, and voriconazole; the inducer of CYP3A4 can be selected from: one or more of aprepitant, barbiturates, bosentan, carbamazepine, efavirenz, felbamate, glucocorticoids, modafinil, nevirapine, oxcarbazepine, phenytoin, phenobarbital, primidone, etravirine, rifampicin, St. John's wort, pioglitazone and topiramate.

### Example 4

The drug-coated balloon was prepared according to the following ratio and method:
1. An CYP3A4 inhibitor (2%), rapamycin (2%), water (5-10%) and ethanol (80-92%) were mixed by weight ratio to obtain a medicinal liquid with rapamycin concentration at 52 mg/ml;
2. The medicinal liquid was loaded into an ultrasonic spraying equipment, and the flow rate was set to 0.01-0.1 ml/min;
3. The rotating speed of the balloon was set to 3 to 5.0 rev/s, and the surface of the balloon 3 was wet with ethanol before spraying;
4. The distance L between the ultrasonic nozzle 2 of the ultrasonic spraying equipment and the balloon 3 was adjusted to 10-30 mm, and the ambient temperature was controlled at 18-28 °C;
5. The ultrasonic spraying equipment was turned on, spraying was performed back and forth 10 times along the axial direction of the balloon 3, and the final drug content on the balloon 3 was controlled to 4.0 ug/mm²;
6. The sprayed balloon 3 was placed in an infrared dryer to dry for 5 minutes.

### Example 5

The drug-coated balloon was prepared according to the following ratio and method:
1. An CYP3A4 inhibitor (4%), rapamycin (1%), water (5-10%) and ethanol (80-92%) were mixed by weight ratio to obtain a medicinal liquid with rapamycin concentration at 52 mg/ml;
2. The medicinal liquid was loaded into an ultrasonic spraying equipment, and the flow rate was set to 0.01-0.1 ml/min;
3. The rotating speed of the balloon was set to 3 to 5.0 rev/s, and the surface of the balloon 3 was wet with ethanol before spraying;
4. The distance L between the ultrasonic nozzle 2 of the ultrasonic spraying equipment and the balloon 3 was adjusted to 10-30 mm, and the ambient temperature was controlled at 18-28 °C;
5. The ultrasonic spraying equipment was turned on, spraying was performed back and forth 10 times along the axial direction of the balloon 3, and the final drug content on the balloon 3 was controlled to 4.0 ug/mm²;
6. The sprayed balloon 3 was placed in an infrared dryer to dry for 5 minutes.

### Example 6

The drug-coated balloon was prepared according to the following ratio and method:
1. An CYP3A4 inducer (3%), rapamycin (3%), water (5-10%) and ethanol (80-92%) were mixed by weight ratio to obtain a medicinal liquid with rapamycin concentration at 52 mg/ml;
2. The medicinal liquid was loaded into an ultrasonic spraying equipment, and the flow rate was set to 0.01-0.1 ml/min;
3. The rotating speed of the balloon was set to 3 to 5.0 rev/s, and the surface of the balloon 3 was wet with ethanol before spraying;
4. The distance L between the ultrasonic nozzle 2 of the ultrasonic spraying equipment and the balloon 3 was adjusted to 10-30 mm, and the ambient temperature was controlled at 18-28 °C;
5. The ultrasonic spraying equipment was turned on, spraying was performed back and forth 10 times along the axial direction of the balloon 3, and the final drug content on the balloon 3 was controlled to 4.0 ug/mm²;
6. The sprayed balloon 3 was placed in an infrared dryer to dry for 5 minutes.

### Example 7

The drug-coated balloon was prepared according to the following ratio and method:
1. An CYP3A4 inducer (4%), rapamycin (1%), water (5-10%) and ethanol (80-92%) were mixed by weight ratio to obtain a medicinal liquid with rapamycin concentration at 52 mg/ml;
2. The medicinal liquid was loaded into an ultrasonic spraying equipment, and the flow rate was set to 0.01-0.1 ml/min;
3. The rotating speed of the balloon was set to 3 to 5.0 rev/s, and the surface of the balloon 3 was wet with ethanol before spraying;
4. The distance L between the ultrasonic nozzle 2 of the ultrasonic spraying equipment and the balloon 3 was adjusted to 10-30 mm, and the ambient temperature was controlled at 18-28 °C;
5. The ultrasonic spraying equipment was turned on, spraying was performed back and forth 10 times along the axial direction of the balloon 3, and the final drug content on the balloon 3 was controlled to 4.0 ug/mm²;
6. The sprayed balloon 3 was placed in an infrared dryer to dry for 5 minutes.

### III. A drug receptor protein inhibitor and a drug metabolism isoenzyme inhibitor were used to prepare the drug-coated balloon controllable in drug metabolism

### Example 8

The drug-coated balloon was prepared according to the following ratio and method:
1. A mixture of a CYP3A4 inhibitor and a FKBP12 inhibitor (2.5%), rapamycin (2.5%), water (5-10%) and ethanol (80-92%) were mixed by weight ratio to obtain a medicinal liquid with rapamycin concentration at 52 mg/ml;
2. The medicinal liquid was loaded into an ultrasonic spraying equipment, and the flow rate was set to 0.01-0.1 ml/min;
3. The rotating speed of the balloon was set to 3 to 5.0 rev/s, and the surface of the balloon 3 was wet with ethanol before spraying;
4. The distance L between the ultrasonic nozzle 2 of the ultrasonic spraying equipment and the balloon 3 was adjusted to 10-30 mm, and the ambient temperature was controlled at 18-28 °C;
5. The ultrasonic spraying equipment was turned on, spraying was performed back and forth 10 times along the axial direction of the balloon 3, and the final drug content on the balloon 3 was controlled to 4.0 ug/mm²;
6. The sprayed balloon 3 was placed in an environment of 18-28 °C and dried for 120 minutes.

In the above examples, a series of drug-coated balloons were prepared by adjusting the types and ratios of the reagents added to the medicinal liquid with rapamycin, and the instant, controllable and slow drug metabolism can be achieved by verification.

Among them, the FKBP12 inhibitor was added in Examples 1, 2, and 3. Since the binding capacity of drug c to FKBP12 protein was stronger than that of drug a, the drug-coated balloons prepared in Examples 2 and 3 had slower drug metabolism than in Example 1. In addition, the ratio of the inhibitor to the drug in Examples 2 and 3 was 50% : 50% and 80% : 20%, respectively. The content of the inhibitor in Example 3 was relatively high, and an extremely slow drug metabolism can be obtained.

The CYP3A4 inhibitor was added in Examples 4 and 5, and the ratio of the CYP3A4 inhibitor to the drug was 50% : 50% and 80% : 20%, respectively. The content of the inhibitor in Example 5 was relatively high, and an extremely slow drug metabolism can be obtained; The CYP3A4 inducer was added in Examples 6 and 7, and the ratio of the CYP3A4 inducer to the drug was 50% : 50% and 80% : 20%, respectively. The content of the inducer in Example 7 was relatively high, which can greatly accelerate the absorption of the drug, and can obtain an extremely fast drug metabolism.

In Example 8, the CYP3A4 inhibitor and the FKBP12 inhibitor were added, and the FKBP12 inhibitor was bound to the rapamycin's receptor protein FKBP12 to inhibit the FKBP12, and at the same time, the CYP3A4 inhibitor inhibited the metabolism of rapamycin by CYP3A4 isoenzyme. The effects of these two aspects work together to further slow down rapamycin metabolism .

Moreover, the drug-coated balloon controllable in drug metabolism prepared in the examples of the present invention can realize immediate drug release after being placed at the site of action, and there is no sustained-release waiting period, and in the process of action, due to the interaction between an inhibitor or an inducer and a protein, there will be no side effects of tearing of the intima caused by balloon inflation. Therefore, the drug-coated balloon of the present invention can achieve a better therapeutic effect and has a significant progress compared with the prior art disclosed in the field.

The preferred embodiments of the present invention have been described in detail above. It should be understood that those of ordinary skill in the art can make many modifications and changes according to the concept of the present invention without creative work. Therefore, any technical solution that can be obtained by logical analysis, reasoning or limited experiments based on the concept of the present invented by a person skilled in the art should be within the scope of protection claimed by the claims.

## Claims

1. A method for preparing a drug-coated balloon controllable in drug metabolism, wherein the method comprises the following steps:
step 1: mixing a drug receptor protein inhibitor, a drug, water, and ethanol to prepare a medicinal liquid;
step 2: loading the medicinal liquid prepared in the step 1 into an ultrasonic spraying equipment, and setting flow parameters and a rotating speed of a balloon;
step 3: adjusting a distance between an ultrasonic nozzle of the ultrasonic spraying equipment and the balloon, controlling an ambient temperature at 18-28 °C, and wetting a surface of the balloon with ethanol before spraying;
step 4: turning on the ultrasonic spraying equipment and spraying back and forth along an axial direction of the balloon; and
step 5: drying the sprayed balloon to obtain the drug-coated balloon.

2. The method for preparing a drug-coated balloon controllable in drug metabolism according to claim 1, wherein the drug receptor protein inhibitor in the step 1 is replaced by a drug metabolism isoenzyme inhibitor or a drug metabolism isoenzyme inducer.

3. The method for preparing a drug-coated balloon controllable in drug metabolism according to claim 2, wherein the drug receptor protein inhibitor in the step 1 is mixed for use with the drug metabolism isoenzyme inhibitor.

4. The method for preparing a drug-coated balloon controllable in drug metabolism according to claim 1, wherein the drug is rapamycin, the drug receptor protein inhibitor is selected from an inhibitor of immunoaffinity protein FKBP12 protein: one or more of 3-pyridin-3-ylpropyl-(2S)-1-(3,3-dimethyl-2-oxo-pentanoyl)-pyrrolidine-2-carboxylat e, (3R)-4-(p-Toluenesulfonyl)-1,4-thiazane-3-carboxylicacid-L-leucine ethyl ester, and (3R)-4-(p-Toluenesulfonyl)-1,4-thiazane-3-carboxylic acid-L-phenyla-lanine benzyl ester.

5. The method for preparing a drug-coated balloon controllable in drug metabolism according to claim 2, wherein the drug is rapamycin, and the drug metabolism isoenzyme inhibitor is selected from an inhibitor of CYP3A4 isoenzyme:
one or more of posaconazole, erythromycin, telithromycin, HIV protease inhibitor, popravir, telaprevir, amiodarone, amprenavir, aprepitant, atonavir, cimetidine, ciprofloxacin, clarithromycin, diltiazem, doxycycline, enoxacin, fluconazole, fluvoxamine, imatinib, indinavir, itraconazole, ketoconazole, miconazole, nefazodone, ritonavir, saquinavir, telithromycin, verapamil, and voriconazole.

6. The method for preparing a drug-coated balloon controllable in drug metabolism according to claim 2, wherein the drug is rapamycin, and the drug metabolism isoenzyme inducer is selected from an inducer of CYP3A4 isoenzyme:
one or more of aprepitant, barbiturates, bosentan, carbamazepine, efavirenz, felbamate, glucocorticoids, modafinil, nevirapine, oxcarbazepine, phenytoin, phenobarbital, primidone, etravirine, rifampicin, St. John's wort, pioglitazone and topiramate.

7. The method for preparing a drug-coated balloon controllable in drug metabolism according to claim 1, wherein a ratio of the drug receptor protein inhibitor to the drug is 0.25-4 : 1, and a drug metabolism slows down as a function of an increase of an addition amount of the drug receptor protein inhibitor.

8. The method for preparing a drug-coated balloon controllable in drug metabolism according to claim 2, wherein a ratio of the drug metabolism isoenzyme inhibitor to the drug is 0.25-4 : 1, and a drug metabolism slows down as a function of an increase of an addition amount of the drug metabolism isoenzyme inhibitor.

9. The method for preparing a drug-coated balloon controllable in drug metabolism according to claim 2, wherein a ratio of the drug metabolism isoenzyme inducer to the drug is 0.25-4 : 1, and a metabolic rate of the drug increases as a function of an increase of an addition amount of the drug metabolism isoenzyme inducer.

10. A drug-coated balloon controllable in drug metabolism prepared according to the method for preparing a drug-coated balloon controllable in drug metabolism according to claim 1, wherein the drug-coated balloon regulates a drug metabolic rate under a condition that a total drug load remains unchanged.

11. A drug-coated balloon controllable in drug metabolism prepared according to the method for preparing a drug-coated balloon controllable in drug metabolism according to claim 2, wherein the drug-coated balloon regulates a drug metabolic rate under a condition that a total drug load remains unchanged.

12. A drug-coated balloon controllable in drug metabolism prepared according to the method for preparing a drug-coated balloon controllable in drug metabolism according to claim 3, wherein the drug-coated balloon regulates a drug metabolic rate under a condition that a total drug load remains unchanged.
